# EUROPÄISCHE PATENTSCHRIFT

(11) **EP 4 085 825 B1**
(45) Veröffentlichungstag und Bekanntmachung des Hinweises auf die Patenterteilung: **04.12.2024**
(21) Anmeldenummer: 22171795.2
(22) Anmeldetag: 05.05.2022
(51) Int. Cl.: A61B 5/022, A61B 5/107

(54) **BLUTDRUCKMESSVERFAHREN MIT OBERARMMANSCHETTE**
BLOOD PRESSURE MEASURING METHOD WITH UPPER ARM CUFF
PROCÉDÉ DE MESURE DE LA PRESSION ARTÉRIELLE POURVU DE BRASSARD

(30) Priorität: 05.05.2021 DE 102021111659
(43) Veröffentlichungstag der Anmeldung: 09.11.2022
(73) Patentinhaber: Beurer GmbH, 89077 Ulm (DE)
(72) Erfinder: BÜHLER, Marco, 89075 Ulm (DE); KÖHLER, Ralf, 89291 Langenau (DE); FORSTNER, Klaus, 71679 Asperg (DE)
(74) Vertreter: Maiwald GmbH

(56) Entgegenhaltungen:
- JP-A- 2009 297 161
- JP-A- 2010 068 922
- US-A1- 2005 245 832
- STERGIOU G S ET AL: "Can an electronic device with a single cuff be accurate in a wide range of arm size? Validation of the Visomat Comfort 20/40 device for home blood pressure monitoring", JOURNAL OF HUMAN HYPERTENSION, NATURE PUBLISHING GROUP, GB, vol. 22, no. 11, 3 July 2008 (2008-07-03), pages 796 - 800, XP037723967, ISSN: 0950-9240, [retrieved on 20080703], DOI: 10.1038/JHH.2008.70

## Beschreibung

Die Erfindung betrifft ein Blutdruckmessverfahren und ein für das Verfahren geeignetes Blutdruckmessgerät.

Die Messung des Blutdrucks am Oberarm mittels einer mit Druckluft gefüllten Manschette ist ein grundsätzlich einfaches und weit verbreitetes Verfahren welches zu wichtigen Informationen über den Gesundheitszustand eines Individuums beiträgt. Es sind allerdings viele Faktoren bekannt, die die Genauigkeit und die Aussagekraft dieser Messung beeinflussen, wie etwa Alter, Geschlecht, Schmerzen, Änderung des Herzrhythmus, Armform oder unpassende Manschettengröße. Für letzteren Faktor wurde in einer Studie herausgefunden, dass bei gleicher Manschette ein 5cm längerer Armumfang zu einer 2-5 mmHg höher gemessenen Systole und zu einer 1-3 mmHg höher gemessenen Diastole führen kann (Fonseca-Reeyes S, de Alba-Garcia JG, Parra-Carillo JZ, Paczka-Zapata JA; Effect of standard cuff on blood pressure readings in patients with obese arms; Blood Press Monit 2003; 8:101-106).

Verfahren zur Blutdruckmessung mit Bestimmung des Oberarm-Umfangs sind aus JP-2009297161-A, JP-2010068922-A, US-2005/245832-A1 und aus STERGIOU G S ET AL: "Can an electronic device with a single cuff be accurate in a wide range of arm size? Validation of the Visomat Comfort 20/40 device for home blood pressure monitoring", JOURNAL OF HUMAN HYPERTENSION, Bd. 22, Nr. 11, Juli 2008, ISSN: 0950-9240, DOI: 10.1038/JHH.2008.70 bekannt.

Es ist daher eine Aufgabe der Erfindung, ein Verfahren vorzuschlagen, das unter Nutzung einfacher Standartmanschetten bzw. unter Nutzung einer einzigen Manschette den Blutdruck misst und dabei eine bessere Messgenauigkeit für verschiedene Oberarmumfänge erreicht.

Zur Lösung der Aufgabe wird mit Anspruch 1 ein Blutdruckmessverfahren an einem Oberarm vorgeschlagen, das Verfahren aufweisend die folgenden Schritte:
- Bereitstellen eines Blutdruckmessgerätes mit einer Manschette;
- Anlegen der Manschette um einen Oberarm eines Patienten;
- Aufpumpen der Manschette und während des Aufpumpens:
- Ermittlung des Aufpumpverhaltens der Manschette anhand der Erfassung mindestens des Integrals der elektrischen Pumpleistung bei vorbestimmter Manschetteninnendruck-Anstiegsrate
- Ermittlung eines Oberarmumfangs des Patienten aus dem Aufpumpverhalten der Manschette, wobei das Aufpumpverhalten der Manschette als Information gespeichert wird und die gespeicherte Information mit einer zuvor von diesem Blutdruckmessgerät und dieser Manschette gespeicherten Information gleicher Art, sofern vorhanden, verglichen und bei Identität oder vorbestimmter Ähnlichkeit einer Person aus einer Personengruppe zugeordnet wird.

Es hat sich überraschend gezeigt, dass das Aufpumpverhalten einer Blutdruckmanschette sich ganz generell mit dem Umfang des Oberarmes vorhersagbar ändert. Die mit der Ermittlung des Aufpumpverhaltens einhergehende automatische Erkennung des Umfangs erlaubt vorteilhafter Weise mit geeigneter, an sich bekannter Software eine geeignete Kalibrationsparametrierung zu wählen, um die Blutdruckmesswerte etwa mittels an sich bekannten Algorithmen oder durch einen einfachen Korrekturfaktor an den tatsächlichen Oberarmumfang anzupassen und dadurch insbesondere für Oberarmen mit großem Armumfang zu verbessern. Es versteht sich, dass der Korrekturfaktor auch geeignet sein kann, für kleine Umfänge des Oberarms eine Verbesserung der Messgenauigkeit zu erreichen.

Abhängig vom Oberarmumfang wird der Blutdruckberechnungsalgorithmus jeweils für die Berechnung der Systole und der Diastole angepasst. Die berechneten Werte können in einem weiteren Verfahrensschritt angezeigt oder ausgegeben werden.

Unter den oben genannten, das Aufpumpverhalten definierenden Parametern hat sich der Druckanstieg pro Zeiteinheit, mithin also die Druckänderungsrate während des Aufpumpvorgangs als besonders geeignet gezeigt. So ist die Druckänderungsrate, d.h. der Druckanstieg in mmHG/s insbesondere in einem Bereich zwischen 40 mmHg und 100 mmHg fast vollkommen linear in Bezug auf den Oberarmumfang. Beispielsweise kann in dem genannten Druckbereich für eine bestimmte Manschette mit bekanntem Aufpumpverhalten bei einem Druckanstieg von 32 mmHg/s ein Oberarmumfang von 23 cm angenommen werden und für einen Druckanstieg von 11 mmHg/s ein Oberarmumfang von 41 cm.

In einem weiteren Aspekt der Erfindung kann auch die Druckänderung während des Aufpumpens für einen vorbestimmten Zeitraum erfasst werden. So kann ein erster Manschettendruck nach 5 Sekunden und ein weiterer Manschettendruck nach 7 Sekunden erfasst werden. Alternativ kann ein erster Manschettendruck nach 9 Sekunden und ein weiterer Manschettendruck nach 11 Sekunden erfasst werden.

In einem weiteren Aspekt der Erfindung wird die Druckänderung während des Aufpumpens in einem vordefinierten Druckbereich, insbesondere in einem Druckbereich zwischen 40 mmHg und 100 mmHg erfasst. In der Praxis kann beispielsweise nach Erreichen des niedrigeren der beiden oben genannten Innendrucke eine erste Zeit und nach Erreichen des anderen, höheren Innendrucks die zweite Zeit erfasst werden. Die Steigung bzw. die Druckänderung pro Zeiteinheit, üblicherweise pro Sekunde, ist der Quotient aus dem Betrag der Druckdifferenz, in dem genannten Fall also 60 mmHg, und dem Betrag der Zeitdifferenz.

Mit der Erfindung wird vorgeschlagen, aus dem Aufpumpverhalten der Manschette den Oberarmumfang zu ermitteln. In einer Weiterbildung kann der ermittelte Oberarmumfang auf einem Display des Blutdruckmessgerätes angezeigt werden.

Hierdurch kann der Oberarmumfang von dem Patienten oder einer anderen Person leicht abgelesen werden und beispielsweise zur Kontrolle der Blutdruckmessung herangezogen werden. Beispielsweise kann ein um einige Zentimeter über früheren Werten liegender angezeigter Oberarmumfang auf einen nicht optimalen Sitz der Manschette am Oberarm hindeuten und dadurch helfen eine fehlerhafte Messung zu korrigieren.

Mit der Erfindung ist ferner vorgesehen, dass das Aufpumpverhalten der Manschette als Information gespeichert wird und die gespeicherte Information mit einer zuvor von diesem Blutdruckmessgerät und dieser Manschette gespeicherten Information gleicher Art, sofern vorhanden, verglichen und bei Identität oder vorbestimmter Ähnlichkeit einer Person aus einer Personengruppe zugeordnet wird. Die Information über das Aufpumpverhalten kann dabei etwa eine Information aus der folgenden Gruppe sein: Oberarmumfang, Druckanstieg in einem bestimmten Zeitintervall, Druckänderung während des Aufpumpens, Manschetteninnendruck nach einer vorbestimmten Aufpumpzeit, Benötigte Aufpumpzeit bis zum Erreichen eines vorbestimmten Manschetteninnendrucks; Integral der elektrischen Pumpleistung bei vorbestimmter Manschetteninnendruck-Anstiegsrate.

Die Speicherung und der Vergleich der genannten Information ermöglicht eine verbesserte Zuordnung der Blutdruckmessung zu einer Person.

Die Speicherung kann entweder am Blutdruckmessgerät selbst oder via einer Internetschnittstelle an sich bekannter Art auf einem externen Server erfolgen.

Ein weiterer Aspekt der Erfindung ist ein maschinenlesbares Medium, etwa in Form einer Speicherkarte mit einem darauf gespeicherten Programmcode , welcher, wenn mittels einer Maschine ausgeführt, das Blutdruckmessverfahren nach der Erfindung bzw. nach mindestens einem der oben genannten Aspekte bzw. Fort- oder Weiterbildungen der Erfindung ausführt.

Ein weiterer Aspekt betrifft ein Blutdruckmessgerät, geeignet zur Durchführung des Blutdruckmessverfahrens, inklusive sämtlicher der zuvor beschriebenen Varianten oder weiteren Aspekte.

Insbesondere kann das Blutdruckmessgerät eine Internetschnittstelle aufweisen, mittels der das durch das Blutdruckmessverfahren erfasste Aufpumpverhalten als Information auf einem Server gespeichert wird.

Die Erfindung kann im Übrigen sowohl für Blutdruckmessgeräte, die eine Blutdruckmessung während des Druckabfalls in der Manschette durchführen, als auch für solche Blutdruckmessgeräte verwendet werden, die eine Blutdruckmessung während des Aufpumpens durchführen. Im letzteren Fall kann die Inflationsrate mittels einem geeigneten Software-Regelalgorithmus konstant gehalten sein. Ein typischer Wert kann beispielsweise zwischen 2 mmHg/s und 15 mmHg/s, bevorzugt zwischen 2mmHG/s und 10 mmHg/s, noch bevorzugt mit 5mmHG/s gewählt sein. Insbesondere kann die Druckanstiegsrate konstant und vom Oberarmumfang unabhängig gewählt sein. In diesem Fall kann die eingebrachte Pumpleistung der Bestimmung des Umfangs dienen. Die Pumpleistung kann etwa als Integral über das pulsweitenmoduierte Signal bei konstanter Signalmplitude definiert sein.

Anhand eines Ausführungsbeispiels soll die Erfindung näher beschrieben werden. Es zeigt
Fig. 1 ein Diagramm zur Steigung des Blutdrucks in einem Bereich von 40 mmHg bis 100 mmHg für verschiedene Armumfänge und
Fig. 2 ein Diagramm mit vier Druckkurven über die Zeit für verschiedene Oberarmumfänge.

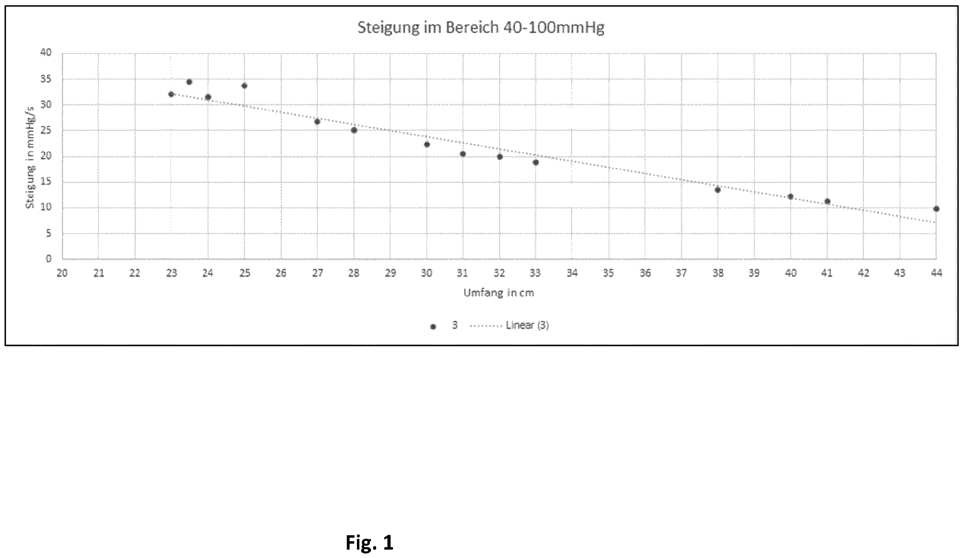

In Fig. 1 ist die Steigung des Blutdrucks in einem Bereich von 40 mmHg bis 100 mmHg für verschiedene Armumfänge gezeigt. Jeder aufgetragene Punkt gibt dabei einen Steigungswert, also den Quotienten aus Druckdifferenz und Zeiteinheit an. Hierfür wurden zuvor 14 Messungen des Blutdrucks vorgenommen, wobei jede Messung an einem anderen bekannten Armumfang erfolgte. Es wurden die Armumfänge in Zentimetern 23; 23,5; 24; 25; 27; 28; 30; 31; 32; 33; 38; 40; 41 für die Erfassung der jeweiligen Steigungsrate (mmHg/s) herangezogen. Im Diagramm wird deutlich, dass insbesondere für größere Armumfänge ein nahezu vollständiger linearer Zusammenhang zwischen der Steigungsrate und dem jeweiligen Armumfang besteht.

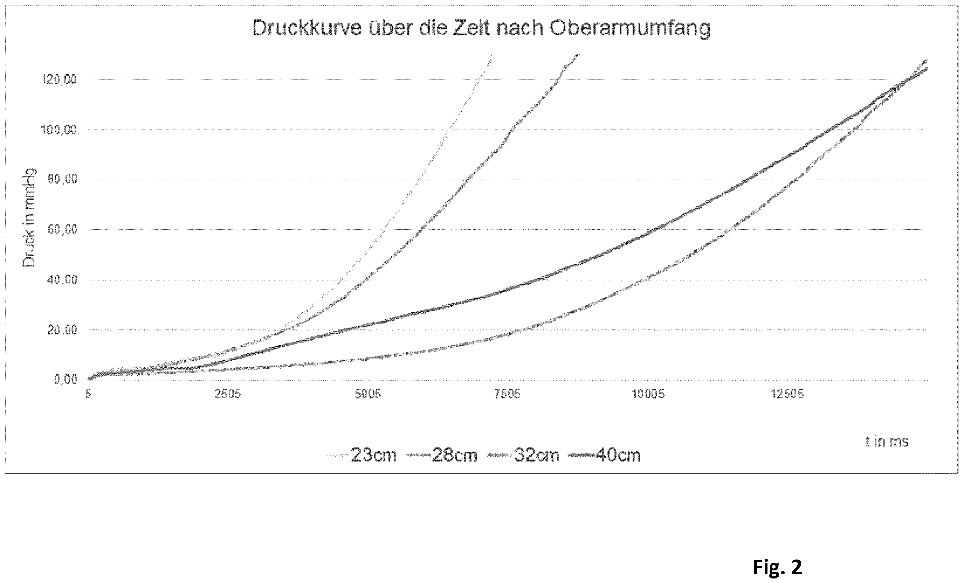

In Figur 2 sind die Druckkurven für die Armumfänge 23, 28, 32 und 40 cm in verschiedenen Graustufen gezeigt. Man erkennt eine mit größerem Armumfang einhergehende geringerer Steilheit bei den gezeigten Kurven. Bemerkenswerter Weise erreicht die 32-cm-Kurve später als die 40-cm-Kurve die 100 mmHg-Marke, also die obere Grenze des in diesem Ausführungsbeispiel betrachteten und für die Bestimmung der Drucksteigungsrate verwerteten Druckbereichs. Gleichwohl ist durch die Betrachtung der Steigungsrate, hier also die Steilheit der Kurve, eine nahezu vollständig lineare Zuordnung zum entsprechenden Oberarmumfang möglich.

Für die oben beschriebenen Messungen wurde eine Manschette in einer üblichen Standart-Ausführung verwendet. Die Manschette ist als einen luftgefüllten Ballon mit Abmessungen in der der Größenordnung von 30 cm x 10 cm x 1 cm ausgebildet, der mit einem nicht dehnbaren Stoff um den Oberarm gewickelt ist. Nach dem Aufblasen bzw. dem automatischen Aufpumpen wird die Außenwand der Manschette starr und die Compliance der Manschette ist ausschließlich auf die darin enthaltene Luft zurückzuführen. Während eines oszillometrischen Durchlaufs wird die Manschette auf einen Druck aufgepumpt, der üblicherweise ganz deutlich über dem systolischen Druck liegt, z.B. 150 mmHg bis 200 mmHg, und dann allmählich mit einer Entlüftungsrate von z.B. 3 mmHg / Sekunde reduziert. Kleine Oszillationen im Manschettendruck entstehen, wenn sich die Arterie mit Blut füllt und entleert, und manifestieren sich als systolischen und diastolischen Druck in der Arterie. Bei einer Anzeige des systolischen und diastolischen Drucks auf einem Display des Blutdruckmessgerätes ist eine den Oberarmumfang berücksichtigende Korrektur der Blutdruckwerte bereits erfolgt.

In einem Aspekt der Erfindung wird somit ein Blutdruckmessgerät vorgeschlagen, dass abhängig von der durch die am Drucksensor gemessene Geschwindigkeit des Druckanstiegs in der Manschette eine Abschätzung des Umfangs des applizierten Körperteils, üblicherweise des Oberarms oder eines Handgelenks, trifft und vorteilhafter Weise durch die Auswahl verschiedener, an sich bekannter Kalibrationsfunktionen oder Kalibrationsprogramme eine höherer Genauigkeit als durch eine feste Kalibration erreicht.

Es versteht sich, dass die Auswahl der Kalibration auch für Bereiche erfolgen kann, die jeweils mehrere Oberarmumfänge abdecken, beispielsweise eine erste Kalibration für einen ersten Bereich für Oberarmumfänge unter 25 cm, eine zweite Kalibration für einen zweiten Bereich für Oberarmumfänge von 25 bis 28 cm und eine dritte Kalibration für einen dritten Bereich für Oberarmumfänge über 28 cm.

Bemerkenswerter Weise eignet sich die Erfassung des Oberarmumfangs auch für eine personale Zuordnung der Blutdruckmessung. Dies gilt insbesondere bei zusätzlicher Berücksichtigung weiterer individueller Faktoren, wie den erfassten Blutdruckwerten selbst.

Im Beispiel der vier in Figur zwei gezeigten Druckkurven könnte beispielsweise jede Druckkurve einem Individuum zugeordnet werden. Für eine vierköpfige Familie, die das erfindungsgemäße Verfahren nutzte, wäre sogar eine fehlerfreie personale Zuordnung möglich ohne weitere individuelle Faktoren zu berücksichtigen, da die Armumfänge durch den zwischen ihnen liegenden größeren Abstand zu signifikanten Änderungen der jeweiligen Drucksteigerungsrate, hier 32 mmHg/s (Kind 1, 5 Jahre) bzw. 25 mmHg/s (Kind 2, 12 Jahre) bzw. 20 mmHg/s (Mutter) bzw. 12 mmHg/s (Großmutter) führen.

## Patentansprüche

1. Blutdruckmessverfahren an einem Oberarm, das Verfahren aufweisend die folgenden Schritte:
- Bereitstellen eines Blutdruckmessgerätes mit einer Manschette;
- Anlegen der Manschette um einen Oberarm eines Patienten;
- Aufpumpen der Manschette und während des Aufpumpens;
- Ermittlung des Aufpumpverhaltens der Manschette anhand der Erfassung mindestens des Integrals der elektrischen Pumpleistung bei vorbestimmter Manschetteninnendruck-Anstiegsrate;
- Ermittlung eines Oberarmumfangs des Patienten aus dem Aufpumpverhalten der Manschette, wobei das Aufpumpverhalten der Manschette als Information gespeichert wird und die gespeicherte Information mit einer zuvor von diesem Blutdruckmessgerät und dieser Manschette gespeicherten Information gleicher Art, sofern vorhanden, verglichen und bei Identität oder vorbestimmter Ähnlichkeit einer Person aus einer Personengruppe zugeordnet wird.

2. Blutdruckmessverfahren nach Anspruch 1, wobei die Druckänderung während des Aufpumpens für einen vorbestimmten Zeitraum oder für einen vordefinierten Druckbereich erfasst wird.

3. Blutdruckmessverfahren nach einem der oben genannten Ansprüche, wobei die Druckänderung während des Aufpumpens in einem vordefinierten Druckbereich, insbesondere in einem Druckbereich zwischen 40 mmHg und 100 mmHg erfasst wird.

4. Blutdruckmessverfahren nach einem der oben genannten Ansprüche, wobei das Blutdruckmessgerät ein Display aufweist und wobei der ermittelte Oberarmumfang auf dem Display angezeigt wird.

5. Blutdruckmessverfahren nach einem der oben genannten Ansprüche, wobei die Information über das Aufpumpverhalten eine Information aus der folgenden Gruppe ist: Oberarmumfang, Druckanstieg in einem bestimmten Zeitintervall, Druckänderung während des Aufpumpens, Manschetteninnendruck nach einer vorbestimmten Aufpumpzeit, Benötigte Aufpumpzeit bis zum Erreichen eines vorbestimmten Manschetteninnendrucks; Integral der elektrischen Pumpleistung bei vorbestimmter Manschetteninnendruck-Anstiegsrate.

6. Maschinenlesbares Medium mit einem darauf gespeicherten Programmcode , welcher, wenn mittels einer Maschine ausgeführt, das Blutdruckmessverfahren nach den Ansprüchen 1 bis 5 durchführt.

7. Blutdruckmessgerät, geeignet zur Durchführung des Blutdruckmessverfahrens nach den Ansprüchen 1 bis 5.

8. Blutdruckmessgerät nach Anspruch 7, wobei das Blutdruckmessgerät eine Internetschnittstelle aufweist, mittels der das durch das Blutdruckmessverfahren erfasste Aufpumpverhalten als Information auf einem Server gespeichert wird.

## Claims

1. Blood pressure measuring method on an upper arm, the method comprising the following steps:
- providing a blood pressure measuring device having a cuff;
- fitting the cuff around an upper arm of a patient;
- inflating the cuff and during inflation;
- determining the inflation behaviour of the cuff on the basis of the detection of at least the integral of the electrical pumping power at a predetermined cuff internal pressure increase rate;
- determining an upper arm circumference of the patient from the inflation behaviour of the cuff, wherein the inflation behaviour of the cuff is stored as information and the stored information is compared with information of the same type, which is previously stored by this blood pressure measuring device and this cuff, if present, and is allocated to a person from a group of persons in the case of identity or predetermined similarity.

2. Blood pressure measuring method according to claim 1,
wherein the pressure change during inflation is detected for a predetermined period of time or for a predefined pressure range.

3. Blood pressure measuring method according to one of the above claims, wherein the pressure change during inflation is detected in a predefined pressure range, in particular in a pressure range between 40 mmHg and 100 mmHg.

4. Blood pressure measuring method according to one of the above claims, wherein the blood pressure measuring device comprises a display, and wherein the determined circumference of the upper arm is displayed on the display.

5. Blood pressure measuring method according to one of the above claims, wherein the information about the inflation behaviour is information from the following group: Upper arm circumference, pressure increase in a certain time interval, pressure change during inflation, cuff internal pressure after a predetermined inflation time, inflation time that is required to reach a predetermined cuff internal pressure; integral of the electrical pumping power at a predetermined cuff internal pressure increase rate.

6. Machine-readable medium having a program code that is stored thereon, which, when executed by means of a machine, implements the blood pressure measuring method according to claims 1 to 5.

7. Blood pressure measuring device, suitable for implementing the blood pressure measuring method according to claims 1 to 5.

8. Blood pressure measuring device according to claim 7,
wherein the blood pressure measuring device comprises an internet interface, by means of which the inflation behaviour that is detected by means of the blood pressure measuring method is stored as information on a server.

## Revendications

1. Procédé de mesure de la pression artérielle sur un bras, le procédé comportant les étapes suivantes :
- mise à disposition d'un appareil de mesure de la pression artérielle doté d'un brassard ;
- placement du brassard autour d'un bras d'un patient ;
- gonflage du brassard et pendant le gonflage ;
- détermination du comportement de gonflage du brassard à l'aide de la détection au moins de l'intégrale de la puissance de pompage électrique à une vitesse d'augmentation prédéterminée de la pression intérieure du brassard ;
- détermination d'une circonférence de bras du patient à partir du comportement de gonflage du brassard, le comportement de gonflage du brassard étant enregistré comme information et l'information enregistrée étant comparée avec une information de même type, si existante, précédemment enregistrée par cet appareil de mesure de la pression artérielle et ce brassard, et, en cas d'égalité ou de ressemblance prédéterminée, associée à une personne d'un groupe de personne.

2. Procédé de mesure de la pression artérielle selon la revendication 1, dans lequel le changement de pression pendant le gonflage est détecté pour un laps de temps prédéterminé ou pour une plage de pression prédéfinie.

3. Procédé de mesure de la pression artérielle selon l'une des revendications précédentes, dans lequel le changement de pression pendant le gonflage est détecté dans une plage de pression prédéfinie, en particulier dans une plage de pression comprise entre 40 mmHg et 100 mmHg.

4. Procédé de mesure de la pression artérielle selon l'une des revendications précédentes, dans lequel l'appareil de mesure de la pression artérielle présente un écran et dans lequel la circonférence de bras déterminée est affichée sur l'écran.

5. Procédé de mesure de la pression artérielle selon l'une des revendications précédentes, dans lequel l'information sur le comportement de gonflage est une information du groupe suivant : circonférence du bras, augmentation de pression dans un intervalle de temps déterminé, changement de pression pendant le gonflage, pression intérieure du brassard après un temps de gonflage prédéterminé, temps de gonflage requis pour atteindre une pression intérieure de brassard prédéterminée ; intégrale de la puissance de pompage électrique à une vitesse d'augmentation prédéterminée de la pression intérieure du brassard.

6. Support lisible par une machine comportant un code de programme enregistré sur celui-ci qui, quand il est exécuté par une machine, exécute le procédé de mesure de la pression artérielle selon les revendications 1 à 5.

7. Appareil de mesure de la pression artérielle, se prêtant à l'exécution du procédé de mesure de la pression artérielle selon les revendications 1 à 5.

8. Appareil de mesure de la pression artérielle selon la revendication 7, dans lequel l'appareil de mesure de la pression artérielle présente une interface Internet au moyen de laquelle le comportement de gonflage détecté par le procédé de mesure de la pression artérielle est enregistré comme information sur un serveur.
